# EUROPEAN PATENT SPECIFICATION

(11) **EP 3 817 616 B1**
(45) Date of publication and mention of the grant of the patent: **07.06.2023**
(21) Application number: 18925114.3
(22) Date of filing: 05.07.2018
(51) Int. Cl.: A43D 1/02, A43B 7/14, A43B 7/28, A61B 5/107

(54) **A METHOD AND A SYSTEM FOR OBTAINING FOOT ANALYSIS DATA**
VERFAHREN UND SYSTEM ZUR GEWINNUNG VON FUSSANALYSEDATEN
PROCÉDÉ ET SYSTÈME D'OBTENTION DE DONNÉES D'ANALYSE DE PIED

(43) Date of publication of application: 12.05.2021
(73) Proprietor: Footbalance System Oy, 01510 Vantaa (FI)
(72) Inventor: HAKKALA, Erkki, 00930 Helsinki (FI)
(74) Representative: Berggren Oy
(86) International application number: PCT/FI2018/050535
(87) International publication number: WO 2020/008101

(56) References cited:
- EP-A1- 2 040 576
- WO-A1-2015/183071
- WO-A1-2018/048880
- US-A1- 2013 174 445
- US-A1- 2013 174 445
- US-A1- 2016 081 435
- US-A1- 2016 081 435
- US-A1- 2016 331 071
- US-A1- 2016 331 071
- US-A1- 2017 255 185
- US-B1- 7 069 665
- US-B1- 7 069 665

## Description

### Field of the Invention

The present invention relates to obtaining foot analysis data.

### Background

Most of the people in the world population suffer from some sort of foot problems. Foot motion/gait problems reflect to soles, ankles, knees, hips, back, etc; that is why their treatment and prevention is particularly beneficial to the whole human well-being. An individual takes around 15 000-16 000 steps every day. The load on feet in sports is many times the weight of the body.

Different (arch) support insoles are available for correcting the foot position. They have been designed to support longitudinal medial and lateral arch but without separately glued wedges they do not actually correct foot position. Wedging is a time-consuming and expensive process. The obtained result depends on the person doing the task and still tends to be rather inaccurate. As another drawback, after gluing the wedges to the soles one cannot take a new mold without first removing the wedges.

Ready-made supports in the insoles do not generally provide a perfect match to anyone's feet, as people do not generally bear identical feet shape. Accordingly, many support insoles are ultimately deemed inconvenient due to their lousy fit. Only few of the people suffering from foot problems have had a chance to purchase insoles that alleviate at least part of the problems. Traditionally, custom-made shoes and insoles have been manufactured by professional shoemakers, physiotherapists, or podiatrists.

Various methods for scanning feet with different kinds of foot scanners have been proposed. However, the scanning methods typically scan images of the sole of the foot, perhaps also providing images from upright position, but where the foot is scanned in the same position. Such scanning method do not adequately take into account the overall biomechanics of the foot and the ankle.

There is, therefore, a need for a solution that improves the overall process of obtaining foot analysis data to be used upon manufacturing of, for example, insoles, custom footwear like shoes and sandals, and various orthotic supports.

US 2013/174445 discloses a foot scanning device with optical means for reading the shape of the foot, said device comprising a plane for standing, a camera arranged to produce a 3D image data about a foot and a computer for storing and processing the image data.

### Summary

Now there has been invented an improved method and technical equipment implementing the method, by which the above problems are alleviated. Various aspects of the invention include a method, and an apparatus which are characterized by what is stated in the independent claims. Various embodiments of the invention are disclosed in the dependent claims.

According to a first aspect, there is provided a method for obtaining foot analysis data for a manufacture of an insole, footwear or an orthopedic support, according to independent claim 1.

According to an embodiment, said second 3D scanning of the shape of said foot is performed, when the person is squatting with a heel in contact with a plane for standing of said 3D scanning apparatus such that a knee angle between a thigh and a leg is substantially between 80 - 150 degrees.

According to an embodiment, the 3D scanning apparatus comprises a plurality of cameras arranged to perform the 3D scanning from an oblique upright direction.

According to an embodiment, the method further comprises performing, by at least one of the cameras, the 3D scanning at least partly from behind the foot.

The method comprises providing, by the cameras of the 3D scanning apparatus, captured 3D image/video data to a computing device for forming a point cloud describing a shape and dimensions of the foot.

According to an embodiment, the method further comprises detecting, based on said at least three 3D scans, whether the foot is subjected to excessive pronation or excessive supination while under pressure.

According to an embodiment, the method further comprises determining, based on foot information on the shape and dimensions of the foot, manufacturing information for forming an insole from an insole preform or for manufacturing footwear, an orthopedic support or at least one pressure sock.

According to a second aspect, there is provided an apparatus for obtaining foot analysis data for a manufacture of an insole, footwear or an orthopedic support, according to independent claim 5.

### Description of the Drawings

In the following, various embodiments of the invention will be described in more detail with reference to the appended drawings, in which
- Fig. 1a: shows a 3D foot scanning device with optical means for reading the shape of the foot according to an embodiment of the invention;
- Fig. 1b: shows a 3D foot scanning device for reading the shape of the foot according to an embodiment of the invention;
- Fig. 2: shows a flow chart of the method for obtaining foot analysis data according to an embodiment of the invention;
- Figs. 3a - 3f: show various positions of a person upon scanning 3D image/video data from his/her foot and the corresponding point clouds formed from the 3D data; and
- Figs. 4a, 4b: show an insole preform to be shaped on the basis of the foot analysis data obtained according to various embodiments of the invention.

### Detailed Description of the Example Embodiments

In the following, several embodiments of the invention will be described in the context of a system for obtaining foot analysis data. The foot analysis data may be utilized, for example, for manufacturing insoles. It is to be noted, however, that the different embodiments have applications in any environment where customized manufacturing from preforms is used. Especially the foot analysis data may be utilized in the manufacturing of footwear, such as shoes and sandals, in a similar manner as manufacturing insoles. Also, different kinds of foot support footwear or orthotic supports, such as foot and ankle supports, orthosis and pressure socks, may be manufactured using the foot analysis data obtained according to the embodiments and may have characteristics similar to insoles created according to the invention. In this line of thought, a shoe may be understood to be any kind of footwear or device intended to be worn on foot. The foot analysis data may further be utilized is selecting an appropriate footwear, for example according to noticed abnormalities in the feet.

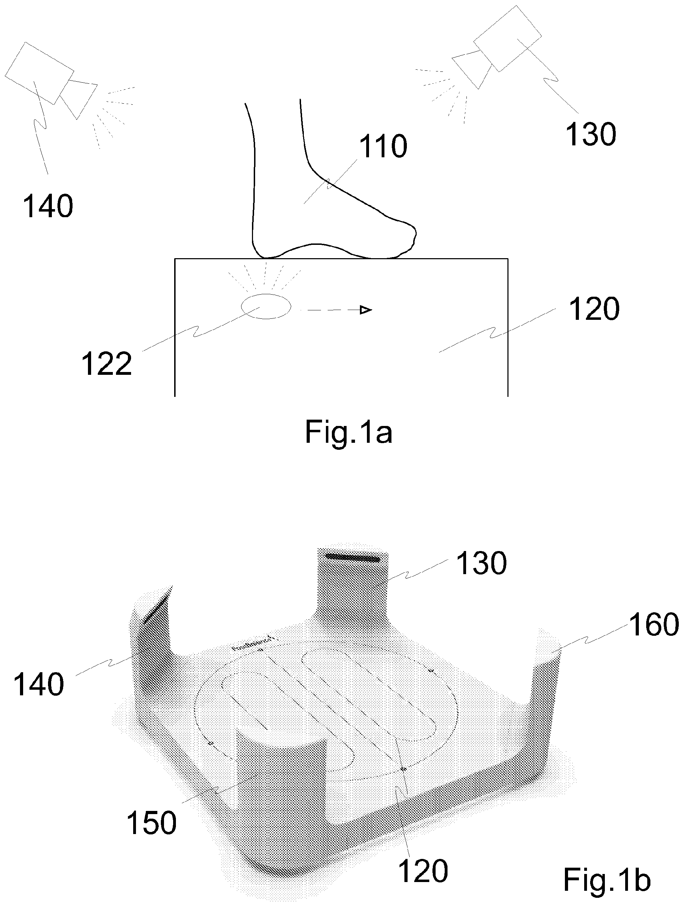

Fig. 1a shows a foot scanning device with optical means for reading the shape of the foot according to an embodiment. The foot scanning arrangement may include, for example, a plane for standing 120, a camera system comprising a plurality of cameras 130, 140, and a computer (not shown).

According to an embodiment, the plurality of cameras 130, 140 are arranged to produce three-dimension (3D) image data about an object, such as a foot. The cameras may be, for example, active 3D cameras (using time-of-flight technology), passive 3D cameras (such as a plenoptic camera), passive sensors such as Kinect or structured light scanners. The camera may be optimized for indoor usage and arranged to obtain exact depth information within a range of less than a few meters, such as Intel^{®} RealSense^{™} Camera SR300 or D435. The 3D image data may also be 2D image data with depth information.

The 3D image data obtained by the plurality of cameras is converted into a point cloud. Point clouds are commonly used data structures for storing 3D content. Point clouds describe the source object, such as a foot, as an set of separate points in the used coordinate values. The volumes of the source objects may be converted to projection surfaces, whereupon the geometry points of a point could of the foot are converted onto a projection surface. The projection surface may surround the source volume (i.e. foot) at least partially such that projection of the geometry primitives is carried out from the centre of the projection surface outwards to the surface. The result is a projection surface describing the contours and the shape of the foot very closely.

The plane for standing may optionally be implemented as a podoscope, which is, by definition, a device for analysing the interaction of the foot and a supporting surface. A person stands on a transparent glass plate of the podoscope, whereupon an image of his feet may be shown through a mirror to the person doing the measurements for manual operation. The foot scanning arrangement, e.g. aforesaid podoscope, may also include data acquisition means such as an optical scanner 122, a camera 130, or some other suitable apparatus for optically and/or electrically imaging the person's feet and their position (and position errors). The imaging of the person's feet may happen from the bottom using a scanner 122 or a camera underneath the glass plate. The imaging may also be done with the help of a camera 130 from the top, and the bottom of the foot may be imaged using a mirror.

Fig. 1b shows a foot scanning device according to an embodiment, where four cameras 130, 140, 150, 160 are arranged to produce 3D image data about at least one foot. The plane for standing 120 is substantially square-shaped, and in each corner of the (rounded) square, there is one camera. Thus, a plurality (i.e. 4 in this case) of cameras are distributed evenly around the object (i.e. at least one foot) such that each camera captures a different 3D image/video representation of the at least one foot. The 3D image/video representations captured by each camera may be input to a computing device for creating a 3D representation of the at least one foot, which 3D representation may further be converted into a point cloud.

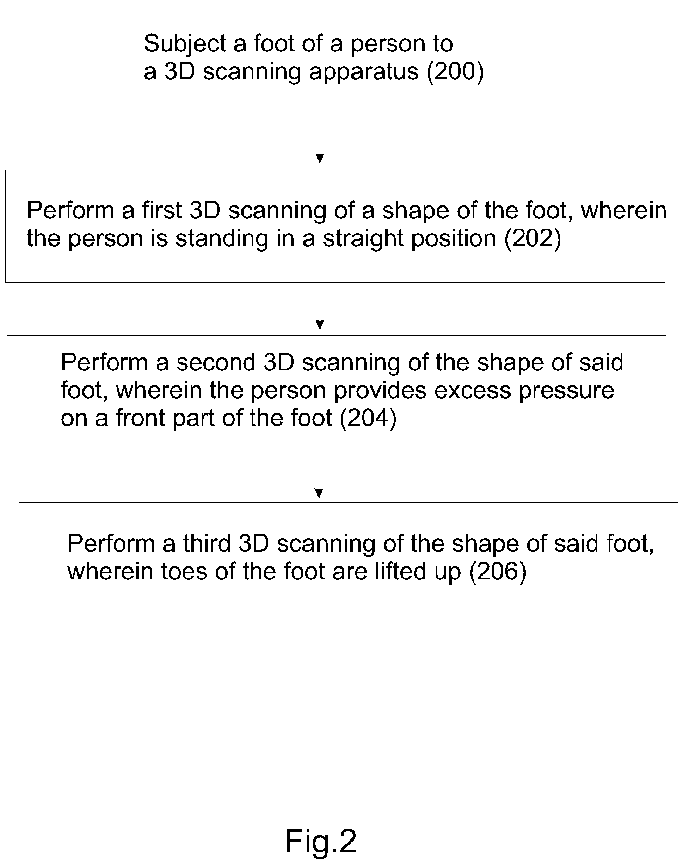

Now, the foot scanning device shown in Figs. 1a and 1b may be utilized in a foot scanning method, which is illustrated in a flow chart of Fig. 2. The method comprises subjecting (200) at least one foot of a person to a three-dimensional (3D) scanning apparatus; performing (202) a first 3D scanning of a shape of the foot, wherein the person is standing in a straight position; performing (204) a second 3D scanning of the shape of said foot, wherein the person provides excess pressure on the foot by squatting; and performing (206) a third 3D scanning of the shape of said foot, wherein toes of the foot are lifted up.

Thus, by carrying out said three 3D scans of the foot of the person, where the person stands on the plane differently for each scanning, the biomechanics of the whole foot may be captured to such detail that any abnormalities or foot motion/gait problems may be detected. For detecting such problems, it may not always be sufficient to scan the bottom surface (i.e. the sole) of the foot, for example with a 2D foot scanner, but it is especially important to determine the operation of the ankle. For obtaining the necessary amount of data for determining the functionality of the ankle, all three scans about the person's foot in different positions are preferably performed.

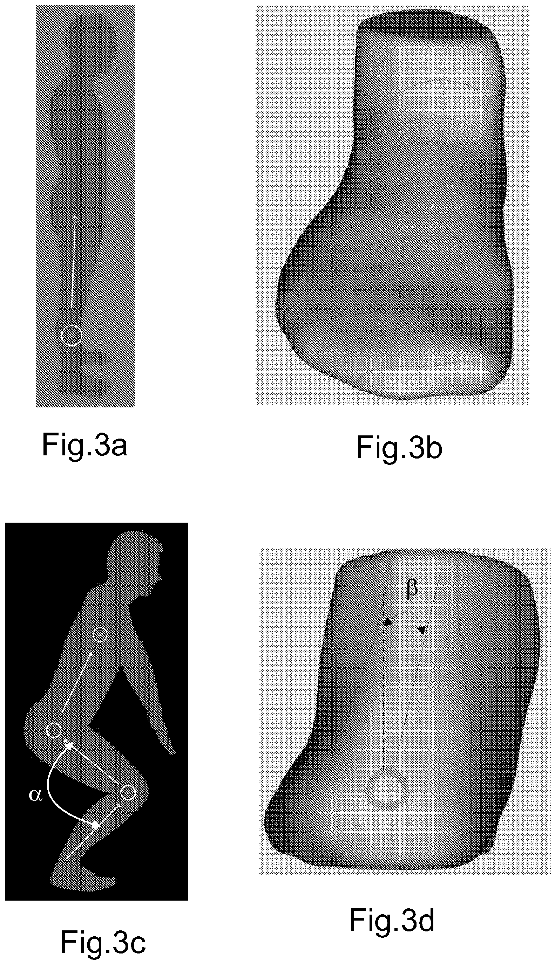

Fig. 3a illustrates the position of the person when performing the first 3D scanning of a shape of the foot. Thus, the person is standing in a straight position. In this position, the weight of the person distributes on the foot as evenly as possible, and the shape and dimensions of the foot may be measured. Fig. 3b illustrates an example of a point cloud captured during the first 3D scanning, shown from frontside of the foot.

According to an embodiment, the method further comprises performing said second 3D scanning of the shape of said foot, when the person is squatting with a heel in contact with a plane for standing of said 3D scanning apparatus such that a knee angle between a thigh and a leg is substantially between 80 - 150 degrees.

Thus, a natural way put more pressure on the foot is to squat. Thereupon, the heel of the foot tends to raise up, but for the purposes of the second 3D scanning, it is important that the heel remains in contact with the plane for standing. The second 3D scanning is preferably carried out when the person is squatting such that a knee angle between a thigh and a leg (a.k.a. shin) is substantially between 80 - 150 degrees. In terms of knee flexion or tibiofemural joint flexion, the same angle range may be denoted as between 30 - 100 degrees .As a result of such squatting, the weight of the person spreads evenly the foot, while inevitably turning the heel bone inwards when looking from behind the foot.

Fig. 3c illustrates the position of the person squatting when performing the second 3D scanning of a shape of the foot. The knee angle between a thigh and a leg is denoted by α.

Fig. 3d illustrates an example of a point cloud captured during the second 3D scanning, shown behind the ankle of the foot. Fig. 3d shows very illustratively how important it is to capture the operation of the ankle under pressure. Upon squatting, the ankle of the foot inevitably turns inwards, as shown by the angle β in Fig. 3d, but in order to detect any abnormalities or problems in the biomechanics of the ankle, it is preferable to measure the shape and dimensions of the foot upon squatting.

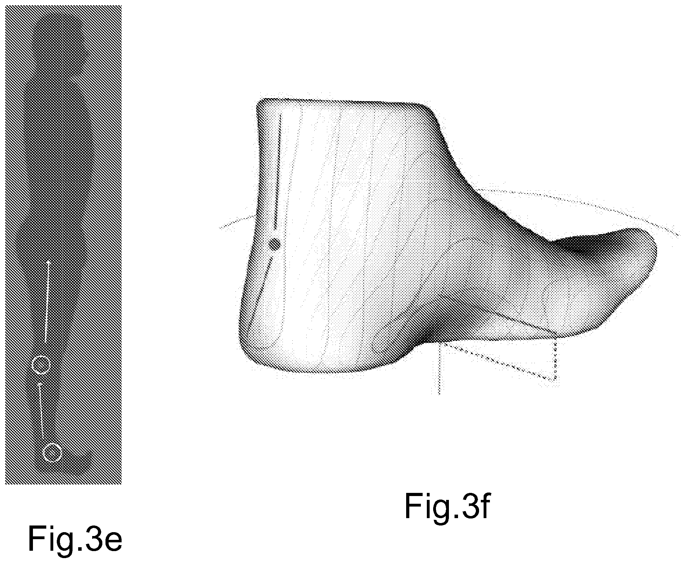

Fig. 3e illustrates the position of the person when performing the third 3D scanning of a shape of the foot. Thus, the person is standing such that s/he lifts his/her toes up. In this position, the so-called Windlass effect takes place, lifting the plantar and transversal arches of the foot higher and straightening the heel bone towards the same line with the shinbone (a.k.a. tibia) when looking from behind the foot, thereby simulating the operation of the foot upon walking or running. The Windlass effect may allow to use the foot's own biomechanics to guide the calcaneus (heel), medial arch and whole foot into a position characteristic to the person. The dorsiflexion of the first metatarsophalangeal joint (big toe) may pull the plantar aponeurosis and shorten the distance between the first metatarsal head and calcaneus. The foot's natural mechanism (Windlass effect) may therefore be used in order to scan the foot in the right position for forming the insoles or footwear.

Fig. 3f illustrates an example of a point cloud captured during the third 3D scanning, shown from right backside of the foot. Again, Fig. 3f shows very illustratively how important it is to capture the operation of the foot by simulating walking or running. By lifting the toes up, any abnormalities or problems in the biomechanics of the foot, especially in the area of plantar arch and/or transversal arch, may be detected, for example, by measuring the height of the plantar and transversal arches of the foot.

According to an embodiment, the 3D scanning apparatus comprises a plurality of cameras arranged to perform the 3D scanning from an oblique upright direction. As shown in Figs. 1a and 1b, the cameras are positioned in such an angle in relation to a centroid and the plane of the object (foot) to be scanned that the shape and the dimensions of the foot can be captured and measures while, at the same time, enabling to capture the operation and biomechanics of the ankle. The angle of the cameras in relation to the plane for standing, when measured from an imaginary centroid of the foot, may be e.g. in range of 15 - 45 degrees. According to an embodiment, at least one of the cameras is arranged to perform the 3D scanning at least partly from behind the foot. As mentioned above, in order to detect any abnormalities or problems in the biomechanics of the ankle or in the area of plantar arch and/or transversal arch of the foot, it is important to have point clouds describing the whole foot and ankle in different positions. For obtaining such point clouds, at least one camera is arranged to perform the 3D scanning at least partly from behind the foot.

The method further comprises detecting, based on said at least three 3D scans, whether the foot is subjected to excessive pronation or excessive supination while under pressure.

Pronation is a natural movement of a human foot, which occurs as weight is transferred from the heel to the forefoot when walking or running and the foot naturally rolls inwards. When standing, pronation occurs as the foot rolls inwards and the arch of the foot flattens. However, if the foot rolls too much inwards, excessive pronation (overpronation) takes place and the arch of the foot flattens too much.

Supination is the natural movement of the foot as it rolls out during the gait cycle. In particular, it is the movement of the subtalar joint (between the talus and calcaneus) into inversion, plantar flexion and adduction. When standing, supination occurs as the foot rolls outwards, placing most of the weight onto the outside of the foot and raising the arch. If the foot rolls too much outwards, excessive supination (oversupination) takes place and the arch of the foot raises too much.

As shown, for example, in Figs. 3d and 3f, the point clouds obtained according to embodiments facilitate significantly detecting problems in foot rolling and e.g. non-average arch heights.

The cameras of the 3D scanning apparatus are configured to provide captured 3D image/video data to a computing device for forming a point cloud describing a shape and dimensions of the foot. Thus, the 3D scanning apparatus may comprise, or be connected to, a computing device such as a PC, a mobile phone, personal digital assistant (PDA), a laptop computer, a computer in the network, a mainframe computer or other computer for image processing. The computing device may then converted the captured 3D image/video data into a point cloud. The computing device may also provide an user interface for the operator, and the user interface may be visual, tactile or audio-based.

The method comprises determining, based on foot information on the shape and dimensions of the foot, manufacturing information for forming an insole from an insole preform. Thus, when the foot information on the shape and dimensions of the person's feet have been measured on the basis of a point cloud, manufacturing information for forming an insole from an insole preform may be determined. The manufacturing information may be linked to the person's identity data and stored in a data system of the insole supplier or manufacturer. Thus, the person may order insoles to be manufactured according his/her personal foot information on the shape and dimensions of his/her feet.

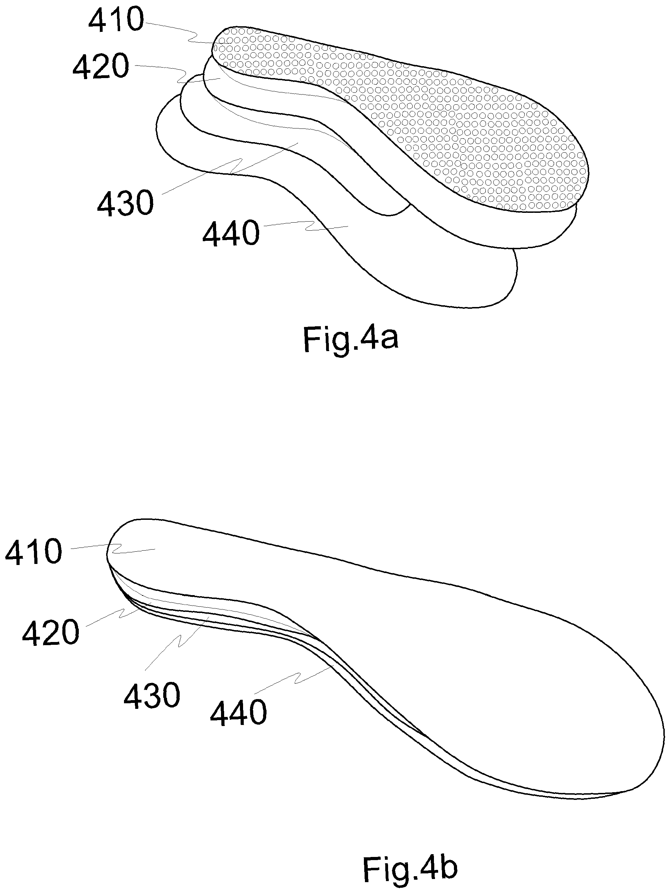

Figs. 4a and 4b show an example of a preform (blank) insole to be shaped based on the foot analysis data obtained according to the embodiments. A preform (blank) insole has at least one layer, which is made of thermoplastic material and reaches out at least from under the heel to under the plantar arch of the target person's foot. Advantageously two, three or four material layers that are connected together are used in the perform insole for comfort. The upper layer 410 of the preform insole is placed against the foot and the lower layer 440 is placed against the shoe. Materials of these two layers can be selected among any common materials used in insoles. For example, the lower layer may be constructed from a known material such as Rheluflex (trademark of Rhenoflex GmbH Ltd) comprising non-woven polyester as a carrier, ionomerresin-ethylvinylacetate blend as an extruded core, and EVA-Hotmelt as an adhesive.

The middle layer 430 of the insole (in case of three layers) is made of thermoplastic. The used thermoplastic can be selected from a large group of known thermoplastics. A significant property for the thermoplastic is the temperature, so-called glass transition temperature, where the thermoplastic becomes plastic and on the other hand turns back to solid form when the temperature is decreasing after shaping the insole. The glass transition temperature is typically lower than polymer melting temperature. Therefore, one good temperature range for shaping the preform may be between the glass transition temperature and the melting temperature. A possible temperature for the thermoplastic to become plastic is preferably somewhere under 95 °C and above 45 °C. Advantageously the range is from 50 °C to 85 °C. The temperature can also be as high as 150 °C Suitable materials that become or are plastic within the preferred ranges are for example thermoplastic polyesters A-PET (Amorphous polyester terephthalate) and PETG (glycol-modified polyethylene terephthalate, which is a copolyester), or such with essentially similar characteristics. Also e.g. ABS (acrylonitrile butadiene styrene), PVC (polyvinyl chloride) can be used. In addition to the first middle layer 830, there may also be another middle layer 420, or even more middle layers than two.

Thickness of the thermoplastic layer may be selected so as to provide reasonable support to the client's foot when the layer is in a rigid state. The thickness may also vary throughout the layer, if e.g. more flexibility is desired under the toe area (thinner) than the plantar arch area (thicker). Other characteristic required for the thermoplastic dictates that it should be rigid under the melting temperature.

When warmed, the material will become flexible and it can be shaped based on the obtained foot analysis data. For example, a pin matrix press comprising a plurality of pins adjustable according to the manufacturing information may be used for shaping the heated preform insole. As another option, 3D printing may be utilized in manufacturing the insole according to the manufacturing information. Herein, a mold with uniform properties (e.g. uniform pressing force) may be used, or a mold with non-uniform properties may be used e.g. to achieve varying thickness.

Insoles and shoes manufactured based on the foot analysis data according to the embodiments may show a better fit to the person's foot than concurrent "custom" insoles or shoes, since the concurrent insoles or shoes aren't often fully customized to the foot, but they are selected as closest match from an existing catalog. The obtained foot analysis data enables to shape the insole or shoe to correct the possible pronation problems. This allows the person to get a corrective insole essentially fully customized to their foot shape.

It needs to be noticed that the techniques described earlier may be used for manufacturing shoes or soles for shoes. The foot may be scanned in a manner similar to manufacturing insoles, and the manufacturing method and apparatuses for creating shoes or soles for shoes may be essentially similar to the method and apparatuses for creating insoles.

The various embodiments of the invention can be implemented with the help of computer program code that resides in a memory and causes the relevant apparatuses to carry out the invention. For example, a manufacturing device and a scanning device may comprise circuitry and electronics for handling, receiving and transmitting data, computer program code in a memory, and a processor that, when running the computer program code, causes the device to carry out the features of an embodiment. Yet further, a remote computer may comprise circuitry and electronics for handling, receiving and transmitting data, computer program code in a memory, and a processor that, when running the computer program code, causes the remote computer to carry out the features of an embodiment.

## Claims

1. A method for obtaining foot analysis data for a manufacture of an insole, footwear, an orthopedic support, or at least one pressure sock, the method comprising:
subjecting at least one foot of a person to a three-dimensional (3D) scanning apparatus;
performing a first 3D scanning of a shape of the foot, wherein the person is standing in a straight position;
performing a second 3D scanning of the shape of said foot, wherein the person provides excess pressure on the foot by squatting; and
performing a third 3D scanning of the shape of said foot, wherein toes of the foot are lifted up;
providing captured 3D image/video data to a computing device for forming a point cloud describing a shape and dimensions of the foot;
detecting, based on said at least three 3D scans, whether the foot is subjected to excessive pronation or excessive supination while under pressure; and
determining, based on the point cloud describing the shape and dimensions of the foot, manufacturing information for forming an insole or for manufacturing footwear, an orthopedic support or at least one pressure sock.

2. The method according to claim 1, wherein said second 3D scanning of the shape of said foot is performed, when the person is squatting with a heel in contact with a plane for standing of said 3D scanning apparatus such that a knee angle between a thigh and a leg is substantially between 80 - 150 degrees.

3. The method according to claim 1 or 2, wherein the 3D scanning apparatus comprises a plurality of cameras arranged to perform the 3D scanning from an oblique upright direction.

4. The method according to claim 3, wherein at least one of the cameras is arranged to perform the 3D scanning at least partly from behind the foot.

5. An apparatus for obtaining foot analysis data for a manufacture of an insole, footwear, an orthopedic support, or at least one pressure sock, said apparatus comprising:
means for subjecting at least one foot of a person to a three-dimensional (3D) scanning apparatus; wherein said 3D scanning apparatus is configured to perform:
- a first 3D scanning of a shape of the foot, wherein the person is standing in a straight position;
- a second 3D scanning of the shape of said foot, wherein the person provides excess pressure on the foot by squatting; and
- a third 3D scanning of the shape of said foot, wherein toes of the foot are lifted up;
wherein said 3D scanning apparatus is configured to provide captured 3D image/video data to a computing device configured to form a point cloud describing a shape and dimensions of the foot;
means for detecting, based on said at least three 3D scans, whether the foot is subjected to excessive pronation or excessive supination while under pressure; and
means for determining, based on the point cloud describing the shape and dimensions of the foot, manufacturing information for forming an insole or for manufacturing footwear, an orthopedic support or at least one pressure sock.

6. The apparatus according to claim 5, wherein
said second 3D scanning of the shape of said foot is configured to be performed when the person is squatting with a heel in contact with a plane for standing of said 3D scanning apparatus such that a knee angle between a thigh and a leg is substantially between 80 - 150 degrees.

7. The apparatus according to claim 5 or 6, wherein the 3D scanning apparatus comprises a plurality of cameras configured to perform the 3D scanning from an oblique upright direction.

8. The apparatus according to claim 7, wherein at least one of the cameras is configured to perform the 3D scanning at least partly from behind the foot.

## Patentansprüche

1. Verfahren zum Erhalten von Fußanalysedaten für die Herstellung einer Einlegesohle, einer Fußbekleidung, einer orthopädischen Stütze oder mindestens eines Druckstrumpfs, wobei das Verfahren Folgendes umfasst:
Aussetzen mindestens eines Fußes einer Person einer dreidimensionalen (3D) Scanvorrichtung;
Durchführen eines ersten 3D-Scannens einer Form des Fußes, wobei die Person in einer geraden Position steht;
Durchführen eines zweiten 3D-Scannens der Form des Fußes, wobei die Person durch Hocken übermäßigen Druck auf den Fuß ausübt; und
Durchführen eines dritten 3D-Scannens der Form des Fußes, wobei die Zehen des Fußes angehoben werden;
Bereitstellen erfasster 3D-Bild-/Videodaten für ein Computergerät zum Bilden einer Punktwolke, die eine Form und Abmessungen des Fußes beschreibt;
Erfassen, basierend auf den mindestens drei 3D-Scans, ob der Fuß einer übermäßigen Pronation oder einer übermäßigen Supination ausgesetzt ist, während er unter Druck steht; und
Bestimmen, basierend auf der Punktwolke, die die Form und die Abmessungen des Fußes beschreibt, Herstellungsinformationen zum Bilden einer Einlegesohle oder zum Herstellen von Fußbekleidung, einer orthopädischen Stütze oder mindestens eines Druckstrumpfs.

2. Verfahren nach Anspruch 1, wobei das zweite 3D-Scannen der Form des Fußes durchgeführt wird, wenn die Person mit einer Ferse in Kontakt mit einer Ebene zum Stehen der 3D-Scanvorrichtung hockt, so dass ein Kniewinkel zwischen einem Oberschenkel und einem Bein im Wesentlichen zwischen 80-150 Grad liegt.

3. Verfahren nach Anspruch 1 oder 2, wobei die 3D-Scanvorrichtung eine Vielzahl von Kameras umfasst, die angeordnet sind, um das 3D-Scannen aus einer schräg aufrechten Richtung durchzuführen.

4. Verfahren nach Anspruch 3, wobei mindestens eine der Kameras angeordnet ist, um das 3D-Scannen mindestens teilweise von hinter dem Fuß durchzuführen.

5. Vorrichtung zum Erhalten von Fußanalysedaten für die Herstellung einer Einlegesohle, einer Fußbekleidung, einer orthopädischen Stütze oder mindestens eines Druckstrumpfs, wobei die Vorrichtung Folgendes umfasst:
Mittel, um mindestens einen Fuß einer Person einer dreidimensionalen (3D) Scanvorrichtung auszusetzen; wobei die 3D-Scanvorrichtung so konfiguriert ist, dass sie Folgendes durchführt:
- ein erstes 3D-Scannen einer Fußform, wobei die Person in einer geraden Position steht;
- ein zweites 3D-Scannen der Form des Fußes, wobei die Person durch Hocken übermäßigen Druck auf den Fuß ausübt; und
- ein drittes 3D-Scannen der Form des Fußes, wobei die Zehen des Fußes angehoben sind;
wobei die 3D-Scanvorrichtung so konfiguriert ist, dass sie erfasste 3D-Bild-/Videodaten an ein Computergerät liefert, das so konfiguriert ist, dass es eine Punktwolke bildet, die eine Form und Abmessungen des Fußes beschreibt;
Mittel zum Erfassen, basierend auf den mindestens drei 3D-Scans, ob der Fuß einer übermäßigen Pronation oder einer übermäßigen Supination ausgesetzt ist, während er unter Druck steht; und
Mittel zum Bestimmen, basierend auf der Punktwolke, die die Form und Abmessungen des Fußes beschreibt, von Herstellungsinformationen zum Bilden einer Einlegesohle oder zum Herstellen von Fußbekleidung, einer orthopädischen Stütze oder mindestens eines Druckstrumpfs.

6. Vorrichtung nach Anspruch 5, wobei
das zweite 3D-Scannen der Form des Fußes konfiguriert ist, um durchgeführt zu werden, wenn die Person mit einer Ferse in Kontakt mit einer Ebene zum Stehen der 3D-Scanvorrichtung hockt, so dass ein Kniewinkel zwischen einem Oberschenkel und einem Bein im Wesentlichen zwischen 80-150 Grad beträgt.

7. Vorrichtung nach Anspruch 5 oder 6, wobei die 3D-Scanvorrichtung eine Vielzahl von Kameras umfasst, die konfiguriert sind, um das 3D-Scannen aus einer schräg aufrechten Richtung durchzuführen.

8. Vorrichtung nach Anspruch 7, wobei mindestens eine der Kameras dazu konfiguriert ist, das 3D-Scannen zumindest teilweise von hinter dem Fuß durchzuführen.

## Revendications

1. Procédé d'obtention de données d'analyse de pied pour la fabrication d'une semelle intérieure, d'un article chaussant, d'un support orthopédique ou d'au moins une chaussette de compression, le procédé comprenant :
la soumission d'au moins un pied d'une personne à un appareil de balayage tridimensionnel (3D) ;
la réalisation d'un premier balayage 3D d'une forme du pied, dans lequel la personne se tient debout dans une position droite ;
la réalisation d'un deuxième balayage 3D de la forme dudit pied, dans lequel la personne exerce une pression excessive sur le pied en s'accroupissant ; et
la réalisation d'un troisième balayage 3D de la forme dudit pied, dans lequel les orteils du pied sont soulevés ;
la fourniture de données d'image/vidéo 3D capturées à un dispositif informatique pour former un nuage de points décrivant une forme et des dimensions du pied ;
le fait de détecter, sur la base desdits au moins trois balayages 3D, si le pied est soumis à une pronation excessive ou à une supination excessive lorsqu'il est sous pression ; et
la détermination, sur la base du nuage de points décrivant la forme et les dimensions du pied, d'informations de fabrication pour former une semelle intérieure ou pour fabriquer un article chaussant, un support orthopédique ou au moins une chaussette de compression.

2. Procédé selon la revendication 1, dans lequel ledit deuxième balayage 3D de la forme dudit pied est réalisé, lorsque la personne est accroupie avec un talon en contact avec un plan pour se tenir debout dudit appareil de balayage 3D de sorte qu'un angle de genou entre une cuisse et une jambe est sensiblement compris entre 80 et 150 degrés.

3. Procédé selon la revendication 1 ou 2, dans lequel l'appareil de balayage 3D comprend une pluralité de caméras agencées pour réaliser le balayage 3D à partir d'une direction verticale oblique.

4. Procédé selon la revendication 3, dans lequel au moins l'une des caméras est agencée pour réaliser le balayage 3D au moins partiellement depuis l'arrière du pied.

5. Appareil d'obtention de données d'analyse de pied pour la fabrication d'une semelle intérieure, d'un article chaussant, d'un support orthopédique ou d'au moins une chaussette de compression, ledit appareil comprenant :
des moyens pour soumettre au moins un pied d'une personne à un appareil de balayage tridimensionnel (3D) ; dans lequel ledit appareil de balayage 3D est configuré pour réaliser :
- un premier balayage 3D d'une forme du pied, dans lequel la personne se tient debout dans une position droite ;
- un deuxième balayage 3D de la forme dudit pied, dans lequel la personne exerce une pression excessive sur le pied en s'accroupissant ; et
- un troisième balayage 3D de la forme dudit pied,
dans lequel les orteils du pied sont soulevés ;
dans lequel ledit appareil de balayage 3D est configuré pour fournir des données d'image/vidéo 3D capturées à un dispositif informatique configuré pour former un nuage de points décrivant une forme et des dimensions du pied ;
des moyens pour détecter, sur la base desdits au moins trois balayages 3D, si le pied est soumis à une pronation excessive ou à une supination excessive lorsqu'il est sous pression ; et
des moyens pour déterminer, sur la base du nuage de points décrivant la forme et les dimensions du pied, des informations de fabrication pour former une semelle intérieure ou pour fabriquer un article chaussant, un support orthopédique ou au moins une chaussette de compression.

6. Appareil selon la revendication 5, dans lequel ledit deuxième balayage 3D de la forme dudit pied est configuré pour être réalisé lorsque la personne est accroupie avec un talon en contact avec un plan pour se tenir debout dudit appareil de balayage 3D de sorte qu'un angle de genou entre une cuisse et une jambe est sensiblement compris entre 80 et 150 degrés.

7. Appareil selon la revendication 5 ou 6, dans lequel l'appareil de balayage 3D comprend une pluralité de caméras configurées pour réaliser le balayage 3D à partir d'une direction verticale oblique.

8. Appareil selon la revendication 7, dans lequel au moins l'une des caméras est configurée pour réaliser le balayage 3D au moins partiellement depuis l'arrière du pied.
